# EUROPEAN PATENT SPECIFICATION

(11) **EP 2 560 951 B1**
(45) Date of publication and mention of the grant of the patent: **25.05.2016**
(21) Application number: 11714776.9
(22) Date of filing: 18.04.2011
(51) Int. Cl.: C07D 207/22, C07D 405/06, A61K 31/401, A61P 3/06

(54) **PRODUCTION OF ATORVASTATIN LOW IN ETHER IMPURITIES**
HERSTELLUNG VON ATORVASTATIN MIT GERINGEN ETHERUNREINHEITEN
PRODUCTION D'ATORVASTATINE À FAIBLE TENEUR EN IMPURETÉS ÉTHERS

(30) Priority: 06.09.2010 EP 10175420; 19.04.2010 EP 10160279
(43) Date of publication of application: 27.02.2013
(73) Proprietor: DSM Sinochem Pharmaceuticals Netherlands B.V., 2613 AX Delft (NL)
(72) Inventor: LANGE, DE, Ben, NL-6151 GE Munstergeleen (NL)
(74) Representative: De Vroom, Erik
(86) International application number: PCT/EP2011/056103
(87) International publication number: WO 2011/131605

(56) References cited:
- WO-A1-2008/075165
- WO-A2-2008/129562

## Description

### Field of the invention

The present invention relates to a method for the production of atorvastatin.

### Background of the invention

Atorvastatin ([*R-(R*,R**)]*-*2*-*(4-fluorophenyl)-β,δ-dihydroxy-5-(1-methylethyl)-3-phenyl-4-[(phenylamino)carbonyl]-1*H*-pyrrole-1-heptanoic acid hemi calcium salt, (2)) is a pharmaceutical ingredient useful as an inhibitor of the enzyme 3-hydroxy-3-methylglutaryl-coenzyme A reductase (HMG-CoA reductase) and thus useful as a hypolipidemic and hypocholesterolemic agent.

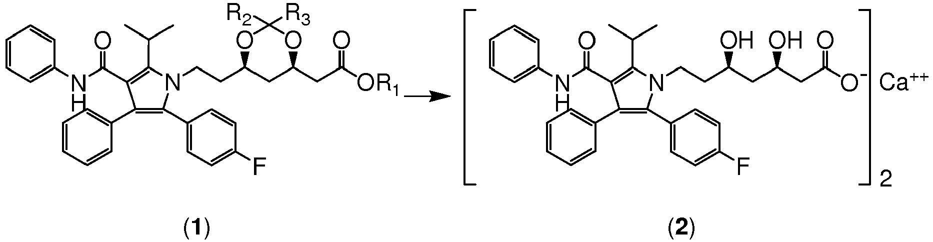

The preparation of atorvastatin is well known and the most common approach is based upon protection/deprotection of the carboxylic acid function as a *tert-butyl* ester ((1); R₁ = -C(CH₃)₃) and of the diol functionality as an acetonide ((1); R₂ = R₃ = -CH₃). This has been described in WO 2008/129562 and in the many references cited therein.

As with any active pharmaceutical intermediate, also for atorvastatin strict control of impurities in the end product is of pivotal importance. The general reaction sequence described above utilizing *tert-*butyl ester protection of the carboxylic acid function has been investigated intensively. The unique chemical nature of the *tert-*butyl ester function has leads to a unique spectrum of impurities, both in terms of chemical structure as well as in terms of relative amounts. Various solutions have been given for reducing the amounts of impurities in the documents mentioned above. However, all these solutions apply to *tert-*butyl ester protection strategies.

Recently, another type of protection has been disclosed in WO 2009/019561, namely the use of the *iso*-propyl group for carboxylic acid protection ((1); R₁ = -CH(CH₃)₂). The differences in chemical reactivity of this group as compared to the *tert-*butyl ester function under the prior art deprotection conditions leads to differences in impurity profiles calling for different solutions.

### Detailed description of the invention

In a first aspect of the invention, the production of atorvastatin hemi calcium salt of formula (2) from a compound of general formula (1) wherein R₁ = -CH(CH₃)₂ and R₂ and R₃ are independently chosen from the list consisting of ethyl, methyl and propyl or wherein R₂ and R₃ form a cyclopentylidene or cyclohexylidene ring, is achieved by the steps of: 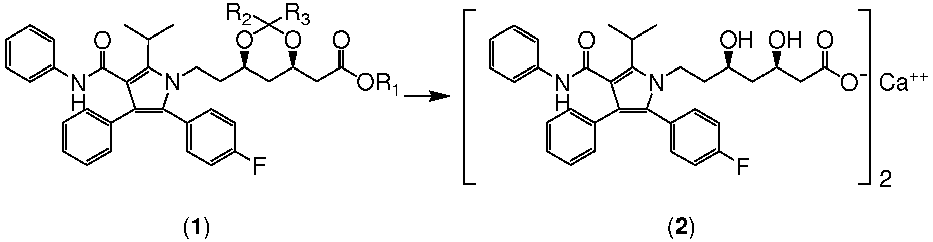
(a) Treating a solution of said compound of general formula (1) in a first solvent with an acid;
(b) Treating the mixture obtained in step (a) with an alkali metal hydroxide;
(c) Treating the mixture obtained in step (b) with a calcium salt or with calcium hydroxide,

Suitable first solvents include acetonitrile, alcohols, cyclic ethers such as dioxane and tetrahydrofuran and ketones such as acetone and mixtures thereof. The first solvent can be an alcohol such as ethanol, *iso*-propanol, methanol and propanol. The acid used in step (a) can be an inorganic acid such as hydrobromic acid, hydrochloric acid, nitric acid, phosphoric acid or sulfuric acid. The amount of alkali metal hydroxide added in step (b) is such that the pH of the mixture is raised to a value between 11 and 13, preferably between 11.5 and 12.5. Suitable alkali metal hydroxides are lithium hydroxide, potassium hydroxide and sodium hydroxide. An example of a calcium salt used in step (c) is calcium acetate. Seed crystals of atorvastatin hemi calcium salt crystal form I may be added prior to the addition of the calcium salt.

Atorvastatin hemi calcium salt precipitates after step (c) and can be isolated by filtration, centrifugation or other techniques known to the skilled person. Optionally the product thus obtained is further purified by re-slurrying in aqueous environment, such as water followed by isolation and drying of the crystals.

In the course of the process impurities (3) and/or (4) are formed.

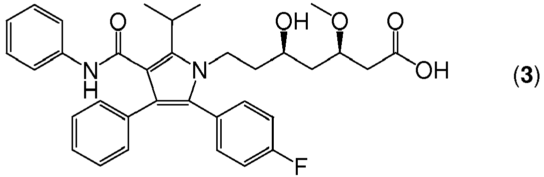

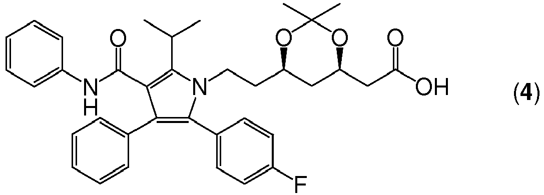

Unfortunately the amounts wherein these impurities are present in the final product may be as high as 0.2% which is unwanted in view of USP and/or European Pharmacopoeia (*Ph. Eur.*) requirements. The levels of these impurities should be below 0.1%. Surprisingly it was found that the amount of impurities could be lowered dramatically by concentrating the mixture obtained in step (a). The best results are obtained when said concentration takes place for a period of from 5 min to 8 h at a temperature of from 10°C to 30°C. The temperature may be maintained below 25ºC. Addition of amounts of the first alcohol during the concentrating process further improves the process so as to allow for prolonged concentration times. By applying the above measure atorvastatin hemi calcium salt crystal form I is obtained with levels of impurities **(3)** and/or **(4)** as low as from 0.0001 % to 0.05%. Concentration may be carried out by evaporation under reduced pressure. Concentration times are from 1 to 4 h, for example from 2 to 3.5 h.

It has been found that in specific cases it may be advantageous to perform a washing step with a second solvent in between process steps (b) and (c). Said second solvent can be an ether such as, for example, dibutyl ether, diethyl ether, methyl ether, methyl *tert-*butylether or an ester such as ethyl acetate or *iso*-propyl acetate or hydrocarbons such as toluene or petroleum ether.

Disclosed herein is a composition comprising atorvastatin hemi calcium salt and from 0.0001 % to 0.05% by weight of a compound of general formula (3) and/or from 0.0001% to 0.05% by weight of a compound of general formula (4). The low amount of impurities as present in the composition of the second aspect is unprecedented and leads to an active pharmaceutical ingredient of increased purity which advantageously leads to lesser side effects during treatment.

### EXAMPLES

### Example 1

### Preparation of atorvastatin calcium from 2-((4R,6R)-6-(2-(3-(phenylcarbamoyl)-5-(4-fluorophenyl)-2-isopropyl-4-phenyl-1H-pyrrol-1-yl)ethyl)-2,2-dimethyl-1,3-dioxan-4-yl)acetic acid isopropyl ester ((1); R₁ = -CH(CH₃)₂, R₂ and R₃ = - CH₃)

2-((4*R*,6*R*)-6-(2-(3-(phenylcarbamoyl)-5-(4-fluorophenyl)-2-isopropyl-4-phenyl-1*H*-pyrrol-1-yl)ethyl)-2,2-dimethyl-1,3-dioxan-4-yl)acetic acid isopropyl ester (20 g, 31 mmol) was added to 600 mL of methanol. The mixture was stirred at 33-35°C until a clear solution was obtained. The solution was cooled to 26-28°C. Then 21 mL of 2.2 N aqueous HCl was added in about 15 min. The reaction was stirred for 7 h at 26-28°C. The mixture was then concentrated under vacuum in 2 h at about 27-29°C to about 50% of its original volume. After concentrating, the reaction mixture was stirred for 30 min. HPLC analysis revealed the starting material to be less than 0.05%. To the mixture, 147 mL of 0.6 N aqueous NaOH was added in 1 h keeping the temperature below 30°C. The pH was about 12. After stirring for 4 h, the clear solution was concentrated under vacuum at 27-29°C until a slurry was obtained. Then 200 mL of water and 100 mL of methyl-*t*-butyl ether were added. The phases were separated. The aqueous phase was treated with 2.0 g active carbon. After filtration of the carbon, the pH of the solution was adjusted to 8.7 with 5% aqueous acetic acid. The reaction mixture was heated until 45-50°C and 40 mL water was added. Next, 2.0 g of atorvastatin calcium polymorph 1 seed was added, followed by a solution of 3.2 g Ca-acetate in 100 mL of water. The mixture was heated to 55-58°C and maintained at this temperature for 30 min. The slurry was cooled to 40-45°C and stirred for 3 h. The solid was isolated by filtration and re-slurried in 200 mL of water. The slurry was heated to 40°C, stirred for 1 h and filtered. The white solid was dried at 50-55°C. Weight 18.0 g. impurities: Ether **(3):** 0.04%; Ketal **(4):** 0.05%.

### Comparative Example 2 (Concentration at 40-45°C)

### Preparation of atorvastatin calcium from 2-((4R,6R)-6-(2-(3-(phenylcarbamoyl)-5-(4-fluorophenyl)-2-isopropyl-4-phenyl-1H-pyrrol-1-yl)ethyl)-2,2-dimethyl-1,3-dioxan-4-yl)acetic acid isopropyl ester ((1); R₁ = -CH(CH₃)₂, R₂ and R₃ = - CH₃)

2-((4R,6R)-6-(2-(3-(phenylcarbamoyl)-5-(4-fluorophenyl)-2-isopropyl-4-phenyl-1*H*-pyrrol-1-yl)ethyl)-2,2-dimethyl-1,3-dioxan-4-yl)acetic acid isopropyl ester (30 g, 31 mmol) was added to 450 mL of methanol. The mixture was stirred at 33-35°C until a clear solution was obtained and then cooled to 26-28°C. Aqueous HCl (2.2 N, 36 mL) was added in 15 min and stirred for 2 h at 26-28°C. The mixture was concentrated under vacuum in 4 h at 40-45°C (about 200 mL was distilled), followed by addition of 200 mL methanol. The reaction mixture was stirred for 1 h, when HPLC analysis revealed the starting material to be less than 0.05%. To the mixture, 207 mL of 0.6 N aqueous NaOH was added in 1 h keeping the temperature below 30°C. The pH was about 12. After stirring for 2 h, the clear solution was concentrated under vacuum at 27-29°C until a slurry was obtained. Water (300 mL) and methyl-*t*-butyl ether (180 mL) were added. The phases were separated. The aqueous phase was treated with 3.0 g active carbon. After filtration of the carbon, the pH of the solution was adjusted to 9.0 with 5% aqueous acetic acid. The reaction mixture was heated to 45-50°C and 60 mL H₂O added. Thereafter, 3.0 g of atorvastatin calcium polymorph I seed was added, followed by addition of a solution of 6.0 g Ca-acetate in 150 mL of water in 1 h. The mixture was heated to 55-58°C and maintained at this temperature for 30 minutes. The slurry was cooled to 40-45°C and stirred for 3 h. The solid was isolated by filtration and re-slurried in 450 mL of water. This slurry was heated to 40°C, stirred for 1 h and filtered. The white solid was dried at 50-55ºC under vacuum. Weight 22.8 g. impurities: Ether **(3):** 0.73%; Ketal **(4):** 0.02%.

### Comparative Example 3 (Without concentration)

### Preparation of atorvastatin calcium from 2-((4R,6R)-6-(2-(3-(phenylcarbamoyl)-5-(4-fluorophenyl)-2-isopropyl-4-phenyl-1H-pyrrol-1-yl)ethyl)-2,2-dimethyl-1,3-dioxan-4-yl)acetic acid isopropyl ester ((1); R₁ = -CH(CH₃)₂, R₂ and R₃ = - CH₃)

2-((4*R*,6*R*)-6-(2-(3-(phenylcarbamoyl)-5-(4-fluorophenyl)-2-isopropyl-4-phenyl-1H-pyrrol-1-yl)ethyl)-2,2-dimethyl-1,3-dioxan-4-yl)acetic acid isopropyl ester (30 g, 31 mmol) was added to 450 mL of methanol. The mixture was stirred at 33-35°C until a clear solution was obtained followed by cooling to 26-28°C. Then 36 mL of 2.2 N aqueous HCl was added in 15 min and the resulting reaction mixture stirred for 2 h at 26-28°C, when HPLC analysis revealed the starting material to be less than 0.2%. To the mixture, 207 mL of 0.6 N aqueous NaOH was added in 1 h keeping the temperature below 30 °C. The pH was about 12. After stirring for 2h, the clear solution was concentrated under vacuum at 27-29°C until a slurry was obtained. Then 300 mL of water and 180 mL of methyl-*t*-butyl ether were added. The phases were separated. Next, the aqueous layer was extracted with a mixture of ethylacetate/cyclohexane (240 mL ethylacetate and 240 mL cyclohexane, 50/50). The phases were separated. Thereafter, the aqueous phase was treated with 3.0 g active carbon. After filtration of the carbon, the pH of the solution was around 8.5. The reaction mixture was heated until 45-50°C and 60 mL H₂O added. 3.0 g of Atorvastatin Calcium Polymorph I seed was added, followed by addition in 1 h of a solution of 6.0 g Ca-acetate in 150 mL of water. The mixture was heated to 55-58°C and maintained at this temperature for 30 minutes. The slurry was cooled to 40-45°C and stirred for 3 h. The solid was isolated by filtration and re-slurried in 400 mL of water. The slurry was heated to 40°C, stirred for 1h and filtered. The white solid was dried at 50-55ºC. Weight 23.6 g. impurities: Ether **(3):** 0.16%; Ketal **(4):** 0.15%.

### Example 4

### Preparation of atorvastatin calcium from 2-((4R,6R)-6-(2-(3-(phenylcarbamoyl)-5-(4-fluorophenyl)-2-isopropyl-4-phenyl-1H-pyrrol-1-yl)ethyl)-2,2-dimethyl-1,3-dioxan-4-yl)acetic acid isopropyl ester ((1); R₁ = -CH(CH₃)₂, R₂ and R₃ = - CH₃)

2-((4*R*,6*R*)-6-(2-(3-(phenylcarbamoyl)-5-(4-fluorophenyl)-2-isopropyl-4-phenyl-1*H*-pyrrol-1-yl)ethyl)-2,2-dimethyl-1,3-dioxan-4-yl)acetic acid isopropyl ester (75 kg, 117 mol) was added to 1125 L of methanol. The mixture was stirred at 35-37°C until clarity. The solution was cooled to 25-27°C. Aqueous HCl solution was added (made from 19 kg concentrated HCl and 64 L of water) and the reaction was stirred for 2 h at 25-27°C. Next, the mixture was concentrated under vacuum in 3.5 h at 20-22°C to ∼50% of its original volume. Then methanol (825 L) was added and the mixture stirred for 45 min. HPLC analysis revealed the starting material to be less than 0.03%. To the mixture aqueous NaOH was added (made from 15 kg NaOH and 560 L water) keeping the temperature below 30°C to give a pH of 12.2. The solution was stirred for 2 h at 25-27°C keeping the pH at not less than 12. The reaction mixture was concentrated to about 900 L under vacuum at a temperature of 20-22°C in 5 h. Next, 750 L of water and 450 L of methyl-*t*-butyl ether were added and stirred for 15 min. The reaction mixture was allowed to settle and the phases were separated. Again 450 L of methyl-*t*-butyl ether was added and stirred for 15 min. The reaction mixture was allowed to settle and the phases were separated. After heating the aqueous layer to 35-37°C, 7.5 kg active carbon was added followed by stirring for 30 min. The reaction mixture was filtered through a Hyflo bed and the carbon/hyflo bed washed with water/methanol (135 L water/15 L methanol). The resulting solution was concentrated under vacuum, followed by addition of 150 L of water and 37.5 L of methyl-*t*-butyl ether. The temperature was increased to 45-47°C and the pH adjusted to 8.6-8.8 with aqueous acetic acid (3L acetic acid in 60 L of water). After heating the reaction mixture until 47-50°C, 7.5 kg of Atorvastatin Calcium Polymorph I seed was added, followed by addition in 1 h of a solution of 14.5 kg Ca-acetate in 375 L of water. The mixture was heated to 55-58°C and maintained at this temperature for 30 min. The slurry was then cooled to 40-45°C and stirred for 3 h. The solid was isolated by centrifugation and the obtained wet-cake re-slurried in 1125 L of water at 40-45°C. After stirring for 1 h, the solid was isolated by centrifugation and dried under vacuum at 50-55°C. Weight 63.5 kg. If required, the material can be milled, blended and/or micronized. Impurities: Ether **(3):** 0.05%; Ketal **(4):** Not Detected.

### Comparative Example 5 (Longer concentration times)

### Preparation of atorvastatin calcium from 2-((4R,6R)-6-(2-(3-(phenylcarbamoyl)-5-(4-fluorophenyl)-2-isopropyl-4-phenyl-1H-pyrrol-1-yl)ethyl)-2,2-dimethyl-1,3-dioxan-4-yl)acetic acid isopropyl ester ((1); R₁ = -CH(CH₃)₂, R₂ and R₃ = - CH₃)

2-((4*R*,6*R*)-6-(2-(3-(phenylcarbamoyl)-5-(4-fluorophenyl)-2-isopropyl-4-phenyl-1*H*-pyrrol-1-yl)ethyl)-2,2-dimethyl-1,3-dioxan-4-yl)acetic acid isopropyl ester (90 kg, 141 mol) was added to 1350 L of methanol. The mixture was stirred at 35-37°C until clarity. The solution was cooled to 25-27°C. Then an aqueous HCl solution was added (made from 22.8 kg concentrated HCl and 77 L of water). The reaction was stirred for 2 h at 25-27°C. The mixture was then concentrated under vacuum in about 16 h at 29-30°C to about 30% of its original volume. Then 825 L of methanol was added and the mixture was again concentrated under vacuum in 5 h at 29-30°C to about 40% of its original volume. To the mixture, 900 L of methanol was charged followed by addition of 620 L of aqueous 0.6 N NaOH keeping the temperature below 30°C to give a pH of not less than 12 (actual temperature 26°C and pH 12.4). The solution was stirred for 2 h at 25-27°C keeping the pH more than 12. The reaction mixture was concentrated (about 900 L was distilled) under vacuum in 8 h at 29-30°C. Next, 810 L of water and 540 L of methyl-*t* butyl ether were added and stirred for 15 minutes. The reaction mixture was allowed to settle and the phases were separated. The aqueous layer was heated to 35-37°C, 5.0 kg active carbon added and stirred for 30 min. The reaction mixture was filtered through a Hyflo bed and the carbon/Hyflo bed washed with water/methanol (80 L water/10 L methanol). To the solution, 22 L of ethylacetate was added and stirred for 1 h. If the pH was not 8.0-8.5, 5 L ethylacetate was added and stirring was continued 1 h more. After heating the reaction mixture to 47-50°C, 9.0 kg of atorvastatin calcium polymorph I seed was added, followed by addition in 1 h of a solution of 14.0 kg Ca-acetate in 270 L of water. The mixture was heated to 55-58°C and maintained at this temperature for 30 minutes. The slurry was then cooled to 40-45°C and stirred for 3 h. The solid was isolated by centrifugation and the wet-cake re-slurried in 900 L of water at 40-45°C. After stirring for 1 h, the solid was isolated by centrifugation and dried under vacuum at 50-55°C. Weight 75.9 kg. If required, the material can be milled, blended and/or micronized. impurities: Ether **(3):** 0.16%; Ketal **(4):** Not Detected.

| **Table: Summary of results** | | | | |
|---|---|---|---|---|
| Example | Concentration | | Impurities | |
| | Temp (°C) | Time (h) | Ether (**3**, %) | Ketal (**4**, %) |
| 1 | 27-29 | 2 | 0.04 | 0.05 |
| 2 | 40-45 | 4 | 0.73 | 0.02 |
| 3 | Not done | | 0.16 | 0.15 |
| 4 | 20-22 | 3.5 | 0.05 | N.D. |
| 5 | 29-30 | 16 | 0.16 | N.D. |

| | | | | |
|---|---|---|---|---|
| N.D. = not detected | | | | |

## Claims

1. Method for the production of atorvastatin hemi calcium salt of formula **(2)** from a compound of general formula **(1)** wherein R₁ = -CH(CH₃)₂ and R₂ and R₃ are independently chosen from the list consisting of ethyl, methyl and propyl or wherein R₂ and R₃ form a cyclopentylidene or cyclohexylidene ring, comprising the steps of: 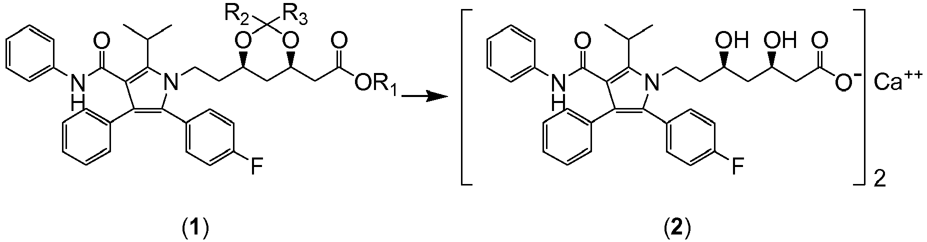
(a) Treating a solution of said compound of general formula (1) in a first solvent with an acid;
(b) Treating the mixture obtained in step (a) with an alkali metal hydroxide;
(c) Treating the mixture obtained in step (b) with a calcium salt or with calcium hydroxide,
**characterized in that** the mixture obtained in step (a) is concentrated for a period of from 5 min to 8 h at a temperature of from 10°C to 30°C.

2. Method according to claim 1 wherein both R₂ and R₃ are methyl.

3. Method according to any one of claims 1 to 2 wherein said first solvent is an alcohol.

4. Method according to any one of claims 1 to 3 wherein said first solvent is methanol.

5. Method according to any one of claims 1 to 4 wherein the mixture obtained after step (b) is washed with a second solvent prior to step (c).

6. Method according to claim 5 wherein said second solvent is an ether.

## Patentansprüche

1. Verfahren zur Herstellung von Atorvastatin-Hemicalciumsalz der Formel (2) aus einer Verbindung der allgemeinen Formel (1), in welcher R₁ = -CH(CH₃)₂ und R₂ und R₃ unabhängig voneinander aus der aus Ethyl, Methyl und Propyl bestehenden Liste ausgewählt sind oder wobei R₂ und R₃ einen Cyclopentyliden- oder Cyclohexylidenring bilden, welches die folgenden Schritte umfasst: 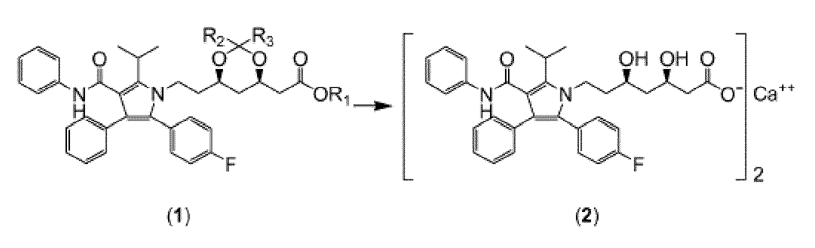
(a) das Behandeln einer Lösung der Verbindung der allgemeinen Formel (1) in einem ersten Lösungsmittel mit einer Säure,
(b) das Behandeln der im Schritt (a) erhaltenen Mischung mit einem Alkalihydroxid,
(c) das Behandeln der im Schritt (b) erhaltenen Mischung mit einem Calciumsalz oder mit Calciumhydroxid,
**dadurch gekennzeichnet, dass** die im Schritt (a) erhaltene Mischung über einen Zeitraum von 5 min bis 8 h bei einer Temperatur von 10°C bis 30°C eingeengt wird.

2. Verfahren nach Anspruch 1, wobei sowohl R₂ als auch R₃ für Methyl stehen.

3. Verfahren nach Anspruch 1 oder 2, wobei es sich bei dem ersten Lösungsmittel um einen Alkohol handelt.

4. Verfahren nach einem der Ansprüche 1 bis 3, wobei es sich bei dem ersten Lösungsmittel um Methanol handelt.

5. Verfahren nach einem der Ansprüche 1 bis 4, bei dem man die nach Schritt (b) erhaltene Mischung vor Schritt (c) mit einem zweiten Lösungsmittel wäscht.

6. Verfahren nach Anspruch 5, wobei es sich bei dem zweiten Lösungsmittel um einen Äther handelt.

## Revendications

1. Procédé de production de sel d'hémi-calcium d'atorvastatine de formule (2) d'un composé de formule générale (1) dans lequel R₁ = -CH(CH₃)₂ et R₂ et R₃ sont indépendamment choisis dans la liste constituée d'éthyle, méthyle et propyle ou dans lequel R₂ et R₃ forment un cycle cyclopentylidène ou cyclohexylidène, comprenant les étapes de : 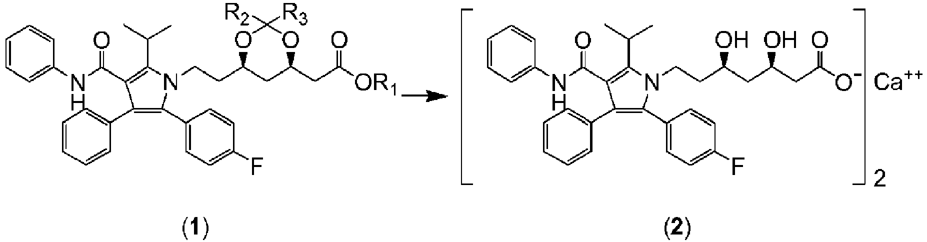
(a) traitement d'une solution dudit composé de formule générale (1) dans un premier solvant avec un acide ;
(b) traitement du mélange obtenu dans l'étape (a) avec un hydroxyde de métal alcalin ;
(c) traitement du mélange obtenu dans l'étape (b) avec un sel de calcium ou avec de l'hydroxyde de calcium, **caractérisé en ce que** le mélange obtenu dans l'étape (a) est concentré pendant une durée de 5 min à 8 h à une température de 10 °C à 30 °C.

2. Procédé selon la revendication 1 dans lequel R₂ et R₃ sont tous deux méthyle.

3. Procédé selon l'une quelconque des revendications 1 à 2 dans lequel ledit premier solvant est un alcool.

4. Procédé selon l'une quelconque des revendications 1 à 3 dans lequel ledit premier solvant est le méthanol.

5. Procédé selon l'une quelconque des revendications 1 à 4 dans lequel le mélange obtenu après l'étape (b) est lavé avec un deuxième solvant avant l'étape (c).

6. Procédé selon la revendication 5 dans lequel ledit deuxième solvant est un éther.
